# EUROPÄISCHE PATENTSCHRIFT

(11) **EP 2 470 266 B1**
(45) Veröffentlichungstag und Bekanntmachung des Hinweises auf die Patenterteilung: **17.12.2014**
(21) Anmeldenummer: 10744936.5
(22) Anmeldetag: 20.08.2010
(51) Int. Cl.: A61N 2/02

(54) **VORRICHTUNG ZUR STIMULIERUNG AUTOREGULATIVER MECHANISMEN DER HOMÖOSTASE DES ORGANISMUS**
Device for stimulating autoregulative mechanisms of the homeostasis in the organism
Dispositif de stimulation de mécanismes autorégulateurs de l'homéostasie de l'organisme

(30) Priorität: 25.08.2009 EP 09168622
(43) Veröffentlichungstag der Anmeldung: 04.07.2012
(73) Patentinhaber: BEMER International AG, 9495 Triesen (LI)
(72) Erfinder: GLEIM, Peter, FL-9495 Schliessa (LI); KLOPP, Rainer, 16348 Wandlitz (DE)
(74) Vertreter: Gulde & Partner
(86) Internationale Anmeldenummer: PCT/EP2010/062162
(87) Internationale Veröffentlichungsnummer: WO 2011/023634

(56) Entgegenhaltungen:
- WO-A1-00/76582
- WO-A1-2008/025731
- WO-A1-2009/090440
- WO-A2-2008/127011
- DE-A1- 10 237 519

## Beschreibung

Die Erfindung betrifft eine Vorrichtung zur Stimulierung autoregulativer Mechanismen der Homöostase des Organismus auf Basis eines pulsierenden elektromagnetischen Feldes.

Es ist bereits bekannt, die Mikrozirkulation durch elektromagnetische Impulse zu beeinflussen.

Aus der EP 0 995 463 ist eine Vorrichtung bekannt, mittels derer biologische Abläufe im menschlichen Körper durch pulsierende elektromagnetische Felder beeinflusst werden, um insbesondere die O₂-Utilisation zu erhöhen und Stoffwechselvorgänge anzuregen. Die Einzelimpulse können dabei einer formelmäßig dargestellten Funktion folgen.

Die WO 2008/025731 beschreibt eine Vorrichtung zur Erzeugung eines gepulsten elektromagnetischen Feldes mit periodischen Impulsen mit an- und absteigenden Hüllkurven in Abhängigkeit von bestimmten Messdaten der Mikrozirkulation des Blutes.

In der WO 00/76582 wird die Ionentransportaktivierung durch Zellmembranen und Kapillarwände mittels niedriggepulsten elektromagnetischen Feldern und stufenförmigen Impulsen beschrieben.

Die WO 2008/127011 beschreibt eine Vorrichtung zur Magnetfeldtherapie mit stufenförmigen Frequenzverläufen mit Frequenzen von 8,7 - 250 Hz und Pulsen im Millisekundenbereich.

Aus der WO 2009/090440 ist eine Diabetesbehandlung mittels gepulstem Magnetfeld bekannt, wobei nur allgemein beschrieben ist, daß die Intensität weniger als 50 µT und die Frequenz 1 Hz bis 1 MHz betragen kann.

Das Gerät zur Magnetfeldtherapie aus DE 102 37 519 A1 erzeugt Frequenzkomponenten von 300 - 1000 Hz mit überlagerten Frequenzen von 2 - 32 Hz, wobei die Intensität von 1,5 bis 150 µT gesteigert werden kann.

Der Erfindung liegt die Aufgabe zugrunde, eine Vorrichtung bereitzustellen, mit der Stimulierungseffekte in der Mikrozirkulation über einen verlängerten Zeitraum erreicht werden.

Eine weitere Aufgabe der Erfindung besteht in der Bereitstellung einer Vorrichtung, mit der Stimulierungseffekte im Bereich der übergeordneten Regulation wie zentrales Nervensystem, insbesondere des vegetativen Nervensystems erreicht werden, vorzugsweise ebenfalls über einen verlängerten Zeitraum.

Erfindungsgemäß umfasst die Vorrichtung einen Impulsgenerator, ein Steuergerät und eine Felderzeugungsvorrichtung, die zusammen ein pulsierendes elektromagnetisches Feld erzeugen, wobei Impulsfolgen mit bestimmten Höhen, bestimmten Abständen und bestimmten Frequenzen die Pulsation des Feldes beeinflussen. Damit können Stimulierungseffekte für autoregulative Mechanismen der Homöostase des Organismus hervorgerufen werden.

Bei der Überlegung, welches morphologische und funktionelle System des Organismus repräsentative Aussagen zu therapierelevanten Wirkungen einer Behandlungsmethode erlaubt, muss dem Transportorgan Blut eine herausragende Bedeutung zugeordnet werden. Die biologische Aufgabe des Organes Blut besteht hauptsächlich in seinen Leistungen zur Homöostase, d.h. der Aufrechterhaltung einer Konstanz des "inneren Milieus" im Rahmen des Zusammenwirkens verschiedener Steuer- und Regelungsvorgänge bei der Realisierung von Fließgleichgewichten sowie seiner Leistungen bei Immunreaktionen.

Die wichtigsten "konstanten Merkmale" des Organismus sind unter physiologischen Bedingungen:
- Wassergehalt
- Zusammensetzung der Körperflüssigkeiten. Blutkonstanten: z.B. pH = 7,3, Gehalt an mineralischen Ionen (Natrium, Kalium, Magnesium, Eisen, Chlorid, Phosphat, Hydrogenkarbonat, Proteingehalt), auch die Glukose-Konzentrationen werden auf einen nur wenig schwankenden Pegel eingeregelt. Erhaltung des Blutvolumens (transkapillärer Flüssigkeitsaustausch, Blutungsstillung, Gerinnung).
- Körpertemperatur des (systemischen) mittleren arteriellen Blutdruckes.

Der lebende Organismus ist ein multistabiles System, in dem verschiedene morphologische Einheiten zur Gewährleistung der Homöostase funktionell durch das Organ Blut verknüpft sind. Diese Einheiten sind äußere Atmung und Stoffwechsel mit dem Herz-Lungen-Kreislauf sowie Nierenfunktion mit der Blutdruckregulierung.

Beim komplizierten Zusammenwirken von Steuer- und Regelungsvorgängen im Organismus mit dem Ziel einer Aufrechterhaltung von Homöostase sind alle Teile des Organismus so miteinander verbunden, dass Änderungen in einem der Teilsysteme (Gewebe, Organ, Organsystem) auf andere Teilsysteme wirken und diese veranlasst, einen neuen Gleichgewichtszustand herzustellen, wobei die Kopplung nerval und durch den Blutkreislauf, u.a. durch die Mikrozirkulation, erfolgt.

Der Funktionszustand eines Organes wird wesentlich durch den Funktionszustand seiner Mikrozirkulation bestimmt. Es ist heute allgemein akzeptiert, dass die meisten Funktionsstörungen bzw. krankhaften Zustände der Organe durch Mikrozirkulationsstörungen wenn nicht sogar ausgelöst, so doch zumindest in ihrem Verlauf determiniert sind. Mikrozirkulationsstörungen entstehen oft im Gefolge von Makrozirkulationsstörungen und können nach und nach eine eigene Dynamik entfalten, die unabhängig vom makrozirkulatorischen Geschehen den Krankheitsverlauf wesentlich beeinflusst oder gar dominiert. Ohne entsprechende Beteiligung der Mikrozirkulation, d.h. der Transportprozesse im Bereich der Mikrogefäße, sind keine Funktionssteigerungen, Abheilungs- oder Restitutionsvorgänge oder Regenerationsvorgänge in den Organen möglich. Wenn überhaupt, so können bei restriktiver Mikrozirkulation bestenfalls vorübergehend in einem geringen Ausmaß Störungs- oder KrankheitsSymptome beeinflusst werden.

Der morphologisch-funktionelle Bereich der Mikrozirkulation lässt daher therapierelevante und prophylaktisch relevante Wirkungen besonders klar erkennen.

Mit der erfindungsgemäßen Vorrichtung wird in erster Linie das Ziel verfolgt, bestimmte Effekte im Bereich der Mikrozirkulation des Blutes hervorzurufen und durch wissenschaftlich bewährte Messmethoden und Messkriterien sichtbar und nachprüfbar zu machen.

Der Erfindung liegt die Aufgabe zugrunde, für lokale Regulationsmechanismen der Mikrozirkulation des Blutes höhere Beträge von Merkmalsänderungen zu erreichen. Eine weitere Aufgabe besteht darin, die Merkmalsänderungen über einen längeren Zeitraum zu erreichen.

Eine weitere Aufgabe besteht darin, über Merkmalsänderungen der Mikrozirkulation des Blutes hinaus, Vorgänge der gesamt-organischen Homöostase-Regulierung positiv zu beeinflussen und damit die körperliche und geistige Leistungsfähigkeit des Menschen zu verbessern.

Bei der erfindungsgemäßen Vorrichtung, die einen Impulsgenerator, ein Steuergerät und eine Felderzeugungseinrichtung umfasst, ist der Impulsgenerator so ausgestaltet, dass er zusammen mit dem Steuergerät und der Felderzeugungseinrichtung ein pulsierendes elektromagnetisches Feld erzeugt durch Abgabe von Impulsfolgen, und die Vorrichtung ist dadurch gekennzeichnet, daß die Impulsfolgen auf einer mit Null beginnenden Zeitachse sind

| | |
|---|---|
| Folge 1: | 0 µT für 2-5 Sekunden, |
| Folge 2: | 3-35 µT Grundsignal für 18-22 Sekunden, |
| Folge 3: | 30-120 µT Zusatzsignal für 100-200 Millisekunden, |
| Folge 4: | zwei- bis dreimalige Wiederholung von Folge 2 und 3, |
| Folge 5: | 0 µT für 2-5 Sekunden nach 60-75 Sekunden auf der Zeitachse, |
| Folge 6: | 3-35 µT Grundsignal für 18-22 Sekunden, |
| Folge 7: | 30-120 µT Zusatzsignal für 100-200 Millisekunden, |
| Folge 8: | 3-35 µT Grundsignal für 18-22 Sekunden, |
| Folge 9: | 30-120 µT Zusatzsignal für 100-200 Millisekunden, |
| Folge 10: | 0 µT für 2-5 Sekunden, |

wobei alle Folgen nach 100 bis 130 Sekunden auf der Zeitachse in der angegebenen Reihenfolge mehrmals wiederholt werden und
wobei die Frequenz in den Folgen 2 und 3 im Bereich von 30-35 Hz liegt und die Frequenz in den Folgen 6 bis 9 im Bereich von 8-15 Hz liegt, das Zusatzsignal in allen Folgen wenigstens 20 µT höher ist als das Grundsignal und die Amplituden der Einzelimpulse einer Exponentialfunktion folgen oder bogenförmig an- und absteigenden Hüllkurven mit harmonischem oder anharmonischem Verlauf.

Die Felderzeugungseinrichtung ist ein Handgerät mit einer Einzelspule und einer relativ kleinen Impulsabgabefläche von unter 100 cm² (Intensivapplikator) oder eine großflächige Spulenmatte zur Ganzkörperbehandlung.

Die Felderzeugungseinrichtung ist vorzugsweise eine Spulenmatte.

Bei einer solchen Spulenmatte können mehrere elektromagnetische Einzelspulen in gleichmäßiger oder ungleichmäßiger Verteilung über die Gesamtfläche der Matte zur Erzeugung eines flächenhaften Magnetfeldes angeordnet sein. Je nach Größe der Matte kann diese auch nur eine einzige Spule enthalten.

Die Einzelspulen sind vorteilhaft als flächeninhaltsgleiche Leiterschleifen oder als Leiterschleifen mit unterschiedlichen Flächen oder als beide Varianten zusammen ausgebildet.

Eine vorteilhafte Ausführungsform der Erfindung besteht darin, dass der Impulsgenerator so ausgestalten ist, dass bei den Impulsfolgen das Zusatzsignal in allen Folgen wenigstens 35 µT höher ist als das Grundsignal, insbesondere wenigstens 45 µT höher als das Grundsignal. Ganz besonders bevorzugt hat das Zusatzsignal eine Intensität von wenigstens 80 µT.

Der Begriff "Grundsignal" im Sinne der Erfindung bedeutet, dass die Maxima der Einzelschwingungen innerhalb der vorgegebenen Zeiteinheit im wesentlichen gleich groß sind und den vorgegebenen Intensitätswert nicht überschreiten, d.h. beispielsweise nicht höher als 35 µT sind.

Das Grundsignal besteht aus einer Folge von Einzelimpulsen mit einer Impulsbreite von ca. 33 ms in der angegebenen Höhe von 3-35 µT, beispielsweise von 15 µT und zwar für den vorgegebenen Zeitraum von 18-22 s. Danach folgt ein Zusatzimpuls mit einer Impulsbreite von 100-200 ms mit einer Intensität (Intensität = Impulsstärke = elektromagnetische Flußdichte) von 30-120 mT, beispielsweise mit 70 µT, also 55 µT höher als der Grundimpuls.

Der Begriff "Zusatzsignal" im Sinne der Erfindung bedeutet somit, dass zu einem bestimmten Zeitpunkt für einen sehr kurzen Zeitraum, hier 100-200 ms, ein deutlich stärkeres Signal mit einer Intensität von wenigstens 20 µT höher als das Grundsignal zusätzlich abgegeben wird, wobei für den kurzen Zeitraum das Grundsignal von dem Zusatzsignal überlagert wird.

Die Impulsfolgen bestehen aus Einzelimpulsen, deren Amplituden z.B. einer Exponentialfunktion folgen. Eine bevorzugte Exponentialfunktion ist in der EP 0995463 B1 beschrieben mit y = x³ · e^{sin(x3)}, wobei die Formel den Verlauf der Amplitude y über die Zeit x angibt. Die Einzelimpulse haben dann einen Verlauf etwa wie in Fig. 2 der EP 0995463 B1. Die Einzelimpulse können auch nicht-exponentielle Verläufe aufweisen, indem sie an- und absteigende Hüllkurven darstellen mit harmonischem oder anharmonischem Schwingungsverlauf wie in der WO 2008/025731. Alternierende Impulsgruppen mit derartigen Schwingungsverläufen sind z.B. in Fig. 4c bis 4f in der WO 2008/025731 wiedergegeben. Insgesamt haben die Impulsgruppen einen bogenförmigen Verlauf.

Impulse oder Impulsgruppen mit stufenweisem oder quadratischem Verlauf sind nicht Gegenstand der Erfindung.

Impulsgruppen mit Einzelimpulsen, deren Amplitude einer e-Funktion entspricht, sind für die vorliegende Erfindung bevorzugt.

Unter dem Begriff "mehrmalige Wiederholung" der Impulsfolgen wird eine 4- bis 10-malige Wiederholung verstanden. In der Wiederholung kann die Zeitdauer des Grundsignals jeweils um 1-2 Sekunden variieren. Die Wiederholung aller Folgen auf der Zeitachse kann vorteilhaft im Bereich von 115-125 Sekunden liegen.

In dem erzeugten elektromagnetischen Feld werden vorteilhaft durch die Höhe des Grundsignals, die Höhe des Zusatzsignals und die Häufigkeit der Wiederholungen der Impulsfolgen funktionelle Merkmale der körpereigenen Regulation des Organismus beeinflusst - ausgewählt unter mikrozirkulatorischen Funktionsmerkmalen, neurovegetativen Merkmalen (insbesondere humorale und nervale Kreislaufregulation), immunologischen Merkmalen und neurologischen Merkmalen.

Zu den mikrozirkulatorischen Funktionsmerkmalen gehören
- die Anzahl der blutzellperfundierten Knotenpunkte (nNP)
- die Änderungen des venulären Strömungsflusses (ΔQᵥₑₙ)
- die Änderungen der venolenseitigen Sauerstoffausschöpfung (ΔpO₂).
- der arterioläre bzw. venuläre Vasomotionszustand (A_{VM}).
Zu den neurovegetativen Merkmalen gehören
- Darm-Motilität (Anzahl der segmentalen Kontraktionsbewegungen in der Zeiteinheit)
- Konzentrationsänderungen endogener Opiatanaloga (Endorphine) im venösen Blut. Zu den immunologischen Merkmalen gehören
- Konzentrationsänderungen von ICAM-1 (intrazelluläres Adhäsionsmolekül)
- Adhäsion weißer Blutzellen an einer definierten Venoleninnenwand-Fläche (nWBC/A).

Zu den Merkmalen der humoralen und zentralnervösen Kreislaufregulation gehören
- Konzentrationsänderungen von Adrenalin/Noradrenalin im Venenblut
- Änderungen der Arteriolendurchmesser in der Subkutis und in der Mucosa des Rectum (kapillarnahe Arteriolen und arteriennahe Arteriolen).

Zu den neurologischen Merkmalen gehören
- Standard-EEG
- Provokations-EEG.

Die mikrozirkulatorischen Funktionsmerkmale betreffend wird bei der Anzahl der aktuell blutzellperfundierten Knotenpunkte in einem definierten mikrovaskulären Netzwerk, nNP, die Anzahl der blutzellperfundierten Verzweigungsorte in diesem Netzwerk als Maß für den Verteilungszustand des Blutes herangezogen. Als Grenzströmungsgeschwindigkeit der roten Blutzellen wird v_{RBC} = 80 µm/s definiert. Die Auswertung erfolgt in + oder - (verglichen mit dem definierten Ausgangswert n=60).

Mittels der vorliegenden Erfindung wird beispielsweise der Wert nNP mit der Impulsfolge 1 - 10 und einer viermaligen Wiederholung der Impulsfolge um 15 - 20 % gegenüber dem Anfangswert erhöht, wobei auch nach 8 Stunden ein höherer Wert als der Anfangswert vorliegt. Damit wird sowohl die unmittelbare Wirkung als auch die Langzeitwirkung der erfindungsgemäßen Stimulierungsvorrichtung deutlich erkennbar.

Der venuläre Strömungsfluss Qᵥₑₙ und der arterioläre Strömungsfluss Qₐᵣₜ ist der Teilchenstrom (Blutzell-Strom) in definierten Venolen bzw. Arteriolen. Er wird in µm³/s angegeben. ΔQᵥₑₙ ist die Änderung des venulären Strömungsflusses und kann im Vergleich mit dem Ausgangswert auch als prozentuale Änderung angegeben werden. Die Werte Qᵥₑₙ und Qₐᵣₜ werden durch die Erfindung deutlich erhöht und dies über einen verlängerten Zeitraum von mehreren Stunden.

Die venolenseitige Sauerstoffausschöpfung ΔpO₂ wird als prozentuale Änderung im Vergleich mit dem jeweiligen Ausgangswert zum Zeitpunkt t=0 angegeben. Bestimmt wird die absolute Differenz der Sauerstoffsättigung des Hämoglobins in den zuführenden Arteriolen und abführenden Venolen des Netzwerkes eines ausgewählten Gewebe-Targets. Als Target werden Gewebeabschnitte von Haut oder Darm ausgewählt, die über ausgedehnte, reich verzweigte und kreislaufrepräsentative Blutgefäß-Netzwerke des Organismus verfügen und ferner zu den immunologisch aktiven Organen zählen, und die weiterhin für nichtinvasive Messungen leicht zugänglich sind. Auch dieser Wert wird durch die Erfindung erhöht und auch über einen verlängerten Zeitraum.

Der spontane arterioläre (bzw. venuläre) Vasomotionszustand, A_{VM}, wird ermittelt, indem das Weg-Zeit-Diagramm der autorhythmischen Kontraktionsbewegungen glatter Muskelzellen der arteriolären Gefäßwand bestimmt wird (Messung der Distanz normal zur Mikrogefäß-Längsachse von Endotheloberfläche zu gegenüberliegender Endotheloberfläche zu äquidistanten Messzeitpunkten; pro Sekunde 60 Messwerte; Ermittlung der zusammengesetzten Schwingung; FOURIER-Analyse; Ermittlung des Amplituden-Frequenz-Spektrums). Kriterium ist der Flächeninhalt A unter der Einhüllenden des Amplituden-Frequenz-Spektrums der arteriolären Vasomotion (eine zusammengesetzte Schwingung). Angegeben wird der Wert als prozentuale Veränderung im Vergleich mit den Ausgangswerten.

Ähnlich wie bei dem Wert nNP wird beispielsweise der mikrozirkulatorische Wert A_{VM} 15 - 25 % erhöht gegenüber dem Grundwert, und er liegt nach 8 Std. noch immer 8 bis 10 % darüber.

Die mit der erfindungsgemäßen Vorrichtung und dem damit erzeugten amplituden- und frequenzmodulierten pulsierenden elektromagnetischen Feld hervorgerufenen Stimulierungseffekte betreffen
1. Lokale Regulationsmechanismen
   der Mikrozirkulation des Blutes, wie die spontane arterioläre Vasomotion, die endothel-vermittelte Tonusregulation, die Bildung und Freisetzung von NO im Endothel
2. Vegetatives Nervensystem
   z.B. Kreislaufwirkungen wie die nervale Ansteuerung der Gefäßlumina-Regulation großkalibriger Arteriolen, die Aktivitäten von Drüsen der inneren Sekretion und Hormongleichgewichte, z.B. durch Konzentrationsänderungen endogener Opiatanaloga (z.B. Endorphine) im venösen Blut; Darm-Motilität (Anzahl segmentaler Kontraktionsbewegungen in der Zeiteinheit)
3. Zentrales Nervensystem und humorale Regelung
   wie Wachheit, Aufmerksamkeit, Konzentrationsfähigkeit; Konzentrationsänderungen von Adrenalin/Noradrenalin im Venenblut.

Mit der Erfindung wird beispielsweise die Konzentration von Adrenalin/Noradrenalin deutlich erhöht.

Weiterhin wird die Konzentration beispielsweise der Endorphine im venösen Blut um 5 - 7 % erhöht im Vergleich zum Ausgangswert, und nach 8 Stunden liegt der erhöhte Wert immer noch 2 - 3 % über dem Ausgangswert. Dies sind signifikante Erhöhungen, die mit anderen Impulsfolgen als denen der Erfindung nicht erreicht werden.

Hinsichtlich der Wirkung bei lokalen Regulationsmechanismen wurde gefunden, dass mittels der erfindungsgemäßen Vorrichtung mit dem erzeugten pulsierenden elektromagnetischen Feld in der ganz speziellen Impulsfolge, Impulshöhe und Impulsdauer gegenüber bekannten elektromagnetischen Feldern sowohl höhere Beträge von Merkmalsänderungen erreicht werden, als auch insbesondere deutlich verlängerte Abklingzeiten. Die Wirksamkeit für einen längeren Zeitraum kann dadurch bis zum 10-fachen verlängert werden.

Dies ist besonders überraschend, weil Impulse mit Grund- und Zusatzsignal im gleichen Frequenzbereich diese Ergebnisse nicht zeigen.

Weiterhin ergeben sich durch die Erfindung prophylaktische und komplementärtherapeutische Wirkungen, die viele Vorgänge der gesamt-organismischen Homöostase-Regulation betreffen. Dies sind beispielsweise gestörte Wundheilung / chronische Wunden, verschiedene therapieresistente chronische Erkrankungen mit Beteiligung der Makro- und Mikrozirkulation, Rehabilitationsmaßnahmen, Steigerung der körperlichen und geistigen Leistungsfähigkeit älterer Menschen, Linderung von Altersbeschwerden, stressbedingte Störungen der Blutperfusion u.a.m., die durch die Erfindung verbessert werden.

Die Wirkungen auf das vegetative und zentrale Nervensystem sind ebenfalls besonders überraschend, da bisherige Magnetfeldbehandlungen mit andersartigen Impulsfolgen dort keine signifikanten Ergebnisse zeigten.

Ein weiterer Gegenstand der Erfindung ist die Bereitstellung eines prophylaktischen oder therapeutischen Verfahrens zur Stimulierung autoregulativer lokaler und übergeordneter Mechanismen der Homöostase des Organismus, dadurch gekennzeichnet, dass der Körper oder ein Teil des Körpers eines Patienten einem pulsierenden elektromagnetischen Feld ausgesetzt wird, bei dem die Impulsfolgen auf einer mit Null beginnenden Zeitachse sind:

| | |
|---|---|
| Folge 1: | 0 µT für 2-5 Sekunden, |
| Folge 2: | 3-35 µT Grundsignal für 18-22 Sekunden, |
| Folge 3: | 30-120 µT Zusatzsignal für 100-200 Millisekunden, |
| Folge 4: | zwei- bis dreimalige Wiederholung von Folge 2 und 3, |
| Folge 5: | 0 µT für 2-5 Sekunden nach 60-75 Sekunden auf der Zeitachse, |
| Folge 6: | 3-35 µT Grundsignal für 18-22 Sekunden, |
| Folge 7: | 30-120 µT Zusatzsignal für 100-200 Millisekunden, |
| Folge 8: | 3-35 µT Grundsignal für 18-22 Sekunden, |
| Folge 9: | 30-120 µT Zusatzsignal für 100-200 Millisekunden, |
| Folge 10: | : 0 µT für 2-5 Sekunden, |

mehrmalige Wiederholung aller Folgen nach 100 bis 130 Sekunden auf der Zeitachse in der angegebenen Reihenfolge über einen Zeitraum von 5-20 Minuten, wobei die Frequenz in den Folgen 2 und 3 im Bereich von 30-35 Hz liegt und die Frequenz in den Folgen 6 bis 9 im Bereich von 8-15 Hz liegt, das Zusatzsignal in allen Folgen wenigstens 20 µT höher ist als das Grundsignal und die Amplituden der Einzelimpulse einer Exponentialfunktion folgen oder an- und absteigenden Hüllkurven mit harmonischem oder anharmonischem Verlauf.

Ein vorteilhafter Zeitraum beträgt 8 - 15 Minuten.

Die Zeitdauer der Behandlung geht von einer einmaligen Anwendung bis zu einer Behandlung von 2 mal pro Tag für 5 - 20 Minuten und für 1-90 Tage, wobei Unterbrechungen von 2-5 Tagen vorgesehen sein können.

Beispielsweise können mit der Spulenmatte 2 Behandlungen von ca. 8 Minuten Dauer im Abstand von 2 Stunden erfolgen. Eine Wiederholung dieser beiden Behandlungen kann alle zwei Tage erfolgen. Es können auch längere oder kürzere Behandlungsintervalls vorgesehen werden in Abhängigkeit von Alter, Konstitution, Kondition und Zustand des zu Behandelnden. Beispiele dafür sind 1 Behandlung von 10 Minuten pro Tag und für 5 Tage hintereinander, dann eine Woche Pause, dann erneut die gleiche Behandlung. Dies kann über 2-3 Monate fortgesetzt werden. Mehrmonatige Behandlungen sind auch mit 2 oder 3 Behandlungen pro Tag, dann 2 oder 3 Tage Pause und erneute 2 oder 3 Behandlungen usw. möglich.

Mit einem kleinflächigen Intensivapplikator können die gleiche Behandlungsdauer oder kürzere Intervalle insbesondere bei höherer magnetischer Flussdichte des Grund- und Zusatzsignals vorgesehen werden, z.B. 30-35 µT für das Grundsignal und 100-120 µT für das Zusatzsignal.

Die Erfindung soll nachstehend durch Beispiele näher erläutert werden. In der dazugehörigen Zeichnung zeigen
- Fig. 1: Diagramm zum Merkmal nNP im Targetgewebe Muscularis Rektum nach Anwendung der erfindungsgemäßen Vorrichtung
- Fig. 2: Diagramm zum Merkmal nNP im Targetgewebe Subkutis nach Anwendung der erfindungsgemäßen Vorrichtung
- Fig. 3: Diagramm zum Merkmal Konzentration der Endorphine c_{E} im Targetgewebe Muscularis Rektum nach Anwendung der erfindungsgemäßen Vorrichtung
- Fig. 4: Diagramm zum Merkmal Konzentration der Endorphine c_{E} im Targetgewebe Subkutis nach Anwendung der erfindungsgemäßen Vorrichtung
- Fig. 5: Diagramm zum Merkmal A_{VM} im Targetgewebe Muscularis Rektum nach Anwendung der erfindungsgemäßen Vorrichtung
- Fig. 6: Diagramm zum Merkmal A_{VM} im Targetgewebe Subkutis nach Anwendung der erfindungsgemäßen Vorrichtung.

### Beispiel 1

Die Vorrichtung besteht aus einem Impulsgenerator, der mit einer Netzspannung von 230 V Wechselstrom / 50 Hz gespeist wird, einer Steuereinrichtung für unterschiedlich hohe Feldstärken an den Applikatoren und einer Spulenmatte als Applikator. Die Betriebsspannung des Steuergerätes und damit die Nennleistung an der Spulenmatte beträgt maximal 12 V Gleichstrom. Eine bevorzugte Vorrichtung ist das Gerät BEMER der Firma Innomed International AG, Liechtenstein. Die Spulenmatte hat eine Fläche von 70x170 cm, so dass der gesamte oder nahezu gesamte liegende Körper eines Patienten darauf Platz hat. In der Spulenmatte sind drei Spulenpaare verteilt. Die elektromagnetische Flußdichte des elektromagnetischen Feldes sowie die zeitlichen Abstände werden über die Steuereinrichtung in mehreren Stufen, z.B. 10 Stufen, gesteuert.

Die Vorrichtung mit dem erfindungsgemäß ausgestalteten Impulsgenerator wird bei einer Probandengruppe getestet. Anzahl der Probanden: 32, aufgeteilt in eine Verum-Gruppe von 16 Probanden und eine Kontrollgruppe von 16 Probanden. Die Probanden waren männlich, Alter 56 - 67 Jahre, stressexponiert.

Die Verum-Gruppe wurde wie folgt behandelt:
- liegend auf der Spulenmatte
- Impulsfolge (zusammengesetzte Schwingungen mit bogenförmigem Verlauf)

| | | | |
|---|---|---|---|
| Folge 1 | 0 µT | 3s | |
| Folge 2 | 35 µT | 18 s; | 33 Hz |
| Folge 3 | 80 µT | 120 ms | |
| Folge 4 | dreimalige Wiederholung von Folge 2 und 3; | | 33 Hz |
| Folge 5 | 0 µT | 3s | |
| Folge 6 | 35 µT | 20 s; | 12 Hz |
| Folge 7 | 80 µT | 120 ms | |
| Folge 8 | 35 µT | 20 s; | 12 Hz |
| Folge 9 | 80 µT | 120 ms | |
| Folge 10 | 0 µT | 2s | |

- Wiederholung der Impulsfolge für insgesamt 10 Minuten Behandlung
- Messung der Merkmale vom Zeitpunkt 0 bis 480 Minuten alle 30 Minuten (äquidistant)
- Probanden dürfen nach der Behandlungszeit aufstehen und einige Schritte auf und ab gehen.

Die Kontrollgruppe wurde wie folgt behandelt:
- liegend auf der Spulenmatte
- Impulsfolge: keine (Placebo) für 10 Minuten
- Messung wie Verum-Gruppe.

Als Messmethoden wurden eingesetzt die Intravitalmikroskopie, Laser-DOPPLER-Mikroflussmessung, Weißlicht-Spektroskopie, intravitalmikroskopische Reflexionsspektroskopie und klinische Labordiagnostik.

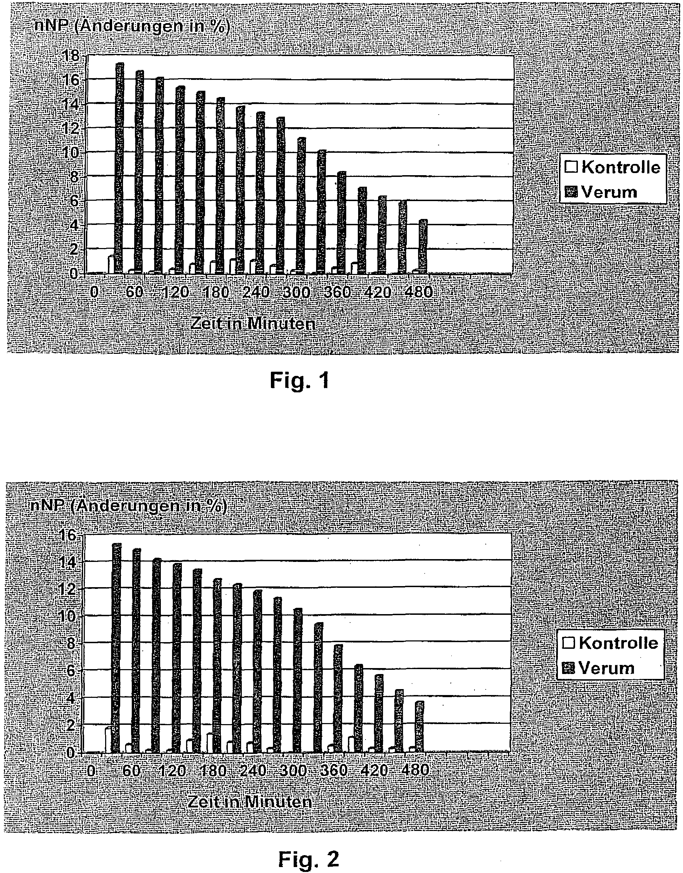

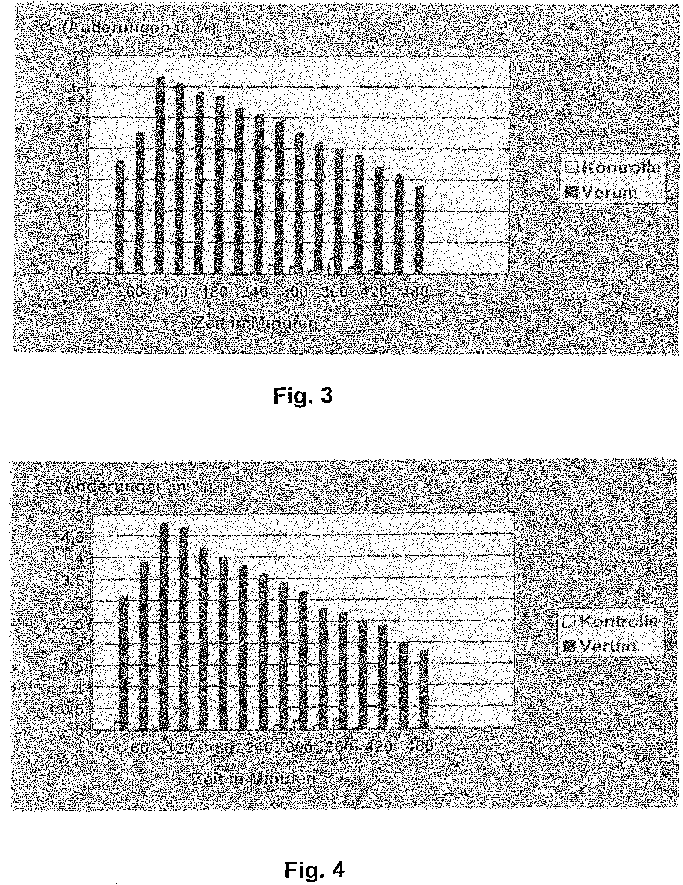

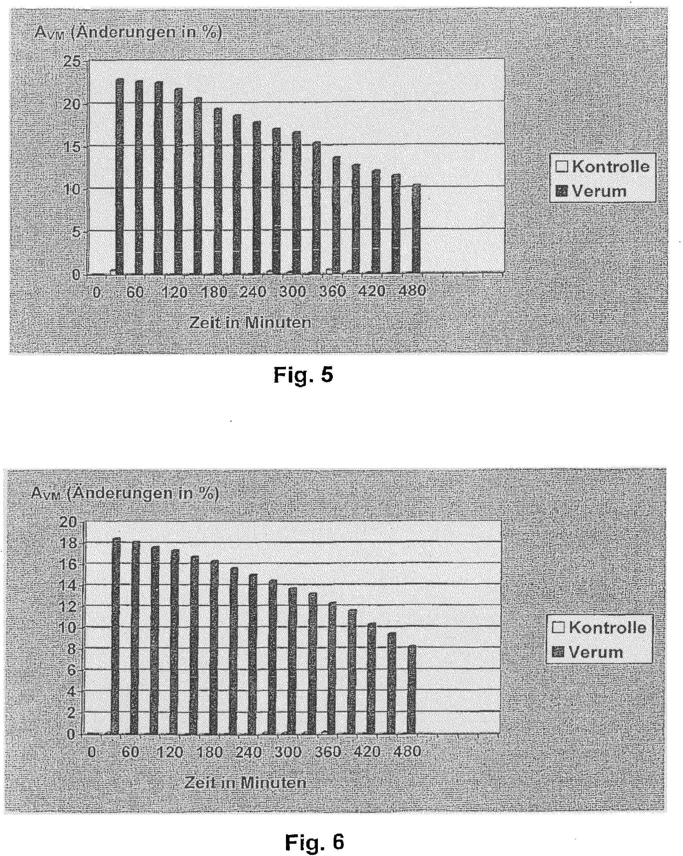

Von den untersuchten Merkmalen wurden hier ausgewählt:
1. Anzahl der blutzellperfundierten Knotenpunkte nNP in einer definierten Gewebeeinheit (V = 2000 µm³) im Targetgewebe Muscularis Rektum und Subkutis.
   Aus der Darstellung in Fig. 1 und Fig. 2 ist zu entnehmen, dass gegenüber der Kontrollgruppe die Verum-Gruppe 30 Minuten nach der Behandlung eine deutlich erhöhte Änderung von 17 % bzw. 15 % zeigte und der Wert der Kontrollgruppe auch nach 8 Stunden noch nicht erreicht war. Es zeigt sich somit eine überraschend lange Abklingquote mit nahezu identischem Verlauf in beiden Gewebearten.
2. Konzentration der Endorphine c_{E} in der Mikrozirkulation einer definierten Gewebevolumeneinheit (V = 2000 µm³) im Targetgewebe Muscularis Rektum und Subkutis
   Aus der Darstellung in Fig. 3 und Fig. 4 ist zu entnehmen, dass gegenüber der Kontrollgruppe bei der Verum-Gruppe nach einem Anstieg auf ein Maximum bei 90 Minuten und einer prozentualen Änderung von über 6 % bzw. knapp 5 % ein sehr langsames Abklingen erfolgt, das am Ende des Untersuchungszeitraumes noch immer deutlich über dem Ausgangswert lag. Auch hier zeigt sich als neurovegetative Merkmalsänderung eine unerwartete Steigerung und eine sehr lange Wirkungszeit. Besonders beeindruckend ist, dass derart gepulste elektromagnetische Felder nicht nur in der Mikrozirkulation Wirkungen zeigen, sondern auch hier z.B. im neurovegetativen System.
3. Flächeninhalt unter der Einhüllenden des originären Amplituden-Frequenzspektrums der spontanen arteriolären Vasomotion A_{VM} in der Mikrozirkulation einer definierten Gewebevolumeneinheit (V = 2000 µm³) im Targetgewebe Muscularis Rektum und Subkutis
   Aus der Darstellung in Fig. 5 und Fig. 6 ist zu entnehmen, dass gegenüber der Kontrollgruppe die Verum-Gruppe 30 Minuten nach der Behandlung eine deutlich erhöhte Änderung von 23 % bzw. 18 % zeigte und auch das Abklingen sehr langsam erfolgte und am Ende des Untersuchungszeitraumes noch immer bei 8 - 10 % lag, so dass erst mehrere Stunden danach mit Erreichen des Ausgangswertes zu rechnen war. Auch hier zeigt sich neben dem unerwartet hohen Änderungsbetrag, der 10 - 15 % über dem zu erwartenden lag, die ebenfalls überraschend lange Abklingzeit der Änderungen.

Insgesamt ergeben sich sowohl für Merkmale der Mikrozirkulation des Blutes als auch für immunologische, neurovegetative, neurologische und Merkmale der zentralnervösen Kreislaufregulation deutliche Veränderungen mittels der erfindungsgemäßen Vorrichtung. Die Beträge sind um 10-15 % höher als zu erwarten und insbesondere ergeben sich signifikant längere Wirksamkeiten durch die Behandlung.

### Beispiel 2 (Vergleichsbeispiel)

Es wird die Vorrichtung der vorliegenden Erfindung mit der Vorrichtung gemäß WO 2008/25731 verglichen.

Es wird mit einer Vorrichtung und einer Spulenmatte wie im Beispiel 1 gearbeitet.

Probanden: männlich, Alter 45 - 55 Jahre, im Rahmen eines physiotherapeutischen Konditionierungsprogrammes.

Verumgruppe 12 Probanden, Vergleichsgruppe 12 Probanden, ausgewählt durch Zufallsgenerator.

Behandlungsintervall 30 Tage (Behandlungen verblindet), Meßintervall ebenfalls 30 Tage (äquidistant und zur gleichen Uhrzeit).
(a) Behandlung der Verumgruppe, jeweils liegend auf der Spulenmatte mit der Impulsfolge (zusammengesetzte Schwingungen mit bogenförmigem Verlauf):

| | | | |
|---|---|---|---|
| Folge 1 | 0 µT | 3 s | |
| Folge 2 | 30 µT | 20 s | 33 Hz |
| Folge 3 | 90 µT | 150 ms | 33 Hz |
| Folge 4 | dreimalige Wiederholung von Folge 2 und 3; | | 33 Hz |
| Folge 5 | 0 µT | 3 s | |
| Folge 6 | 25 µT | 18 s | 12 Hz |
| Folge 7 | 70 µT | 120 ms | 12 Hz |
| Folge 8 | 25 µT | 20 s | 12 Hz |
| Folge 9 | 70 µT | 120 ms | 12 Hz |
| Folge 10 | 0 µT | 2 s | |

- Wiederholung der Impulsfolge für insgesamt 15 Minuten Behandlung
- 2 Stunden Pause
- nochmalige Wiederholung der Impulsfolgen wie oben für 15 Minuten.
Messungen erfolgen an den Tagen 0, 5, 10, 15, 20, 25 und 30.
(b) Behandlung der Vergleichsgruppe, jeweils liegend auf der Spulenmatte mit der Impulsfolge gemäß WO2008/025731 (zusammengesetzte Schwingungen mit bogenförmigem Verlauf):

| | |
|---|---|
| Basisimpuls: | 60 µT, Impulsbreite 30 ms |
| Zusatzimpuls: | 180 µT, Impulsbreite 150 ms |
| Frequenz des Zusatzimpulses: | 3 pro Minute |
| Gesamtfrequenz: | 30 Hz |
| Behandlungsdauer: | 2 x 25 Minuten im Abstand von 2 h |
| Behandlungsfolge: | täglich für 30 Tage |
| Messungen an den Tagen 0, 5, 10, 15, 20, 25, 30 | |

(c) Auswertungsmethoden
Vitalmikroskopische Untersuchungseinheit mit computergestützter Bildverarbeitung (System KONTRON), vitalmikroskopische Reflexionsspektometrie (System SPEX), kombinierte Laser-Doppler-Mikroflußmessung und Weißlicht-spektroskopie (System LEA).
(d) Auswertungsmerkmale
- Spontane arterioläre Vasomotion AVM (Flächeninhalt unter der Einhüllenden des originären Amplituden-Frequenzspektrums der kleinkalibrigen arteriolären Vasomotion),
- Anzahl der aktuell blutzellperfundierten Knotenpunkte im definierten Kapillarnetzwerk nNP,
- venolenseitige Sauerstoffausschöpfung ΔpO₂
- Strömungsfluß der initialen Lymphe QL.
Targetgewebe: Intestinum (Muscularis des Rectum).
Biometrie :
WILCOXON-Rangsummentest (α = 5 %)

**Tabelle 1**

| Spontane arterioläre Vasomotion AVM | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 14,8 | 20,9 | 24,6 | 25,1 | 25,4 | 25,8 |
| Vergl.bsp. 2(b) WO2008/025731 | - | 6,2 | 14,6 | 15,2 | 15,5 | 15,6 | 15,6 |

**Tabelle 2**

| Anzahl der blutzellperfundierten Knotenpunkte nNP | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 9,8 | 15,5 | 21,0 | 25,7 | 26,8 | 27,2 |
| Vergl.bsp. 2(b) WO2008/025731 | - | 4,9 | 9,6 | 13,9 | 15,2 | 15,6 | 15,7 |

**Tabelle 3**

| Venolenseitige Sauerstoffausschöpfung ΔpO₂ | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 9,4 | 15,1 | 20,9 | 25,6 | 27,7 | 29,2 |
| Vergl.bsp. 2(b) WO2008/025731 | - | 5,0 | 8,8 | 14,4 | 15,8 | 18,1 | 18,3 |

**Tabelle 4**

| Strömungsfluß der initialen Lymphe QL | | | | | | | |
|---|---|---|---|---|---|---|---|
| | Änderung in % nach Tagen | | | | | | |
| | 0 | 5 | 10 | 15 | 20 | 25 | 30 |
| Vergl.bsp. 2 (a) ERFINDUNG | - | 18,1 | 23,0 | 29,3 | 32,1 | 36,3 | 38,6 |
| Vergl.bsp. 2(b) WO2008/025731 | - | 4,5 | 7,9 | 9,9 | 11,6 | 14,8 | 15,2 |

Der Vergleich zeigt signifikante Unterschiede für alle vier gemessenen Merkmale der Mikrozirkulation des Blutes zwischen der Vorrichtung des Standes der Technik WO 2008/025731 und der der Erfindung. Das Merkmal QL liegt teilweise um das 2-bis 3-fache höher, andere Merkmale um das 1,5- bis 2-fache.

## Patentansprüche

1. Vorrichtung zur Stimulierung autoregulativer Mechanismen der Homöostase des Organismus, umfassend einen Impulsgenerator, ein Steuergerät und eine Felderzeugungseinrichtung, wobei der Impulsgenerator ausgestaltet ist, zusammen mit dem Steuergerät und der Felderzeugungseinrichtung ein pulsierendes elektromagnetisches Feld zu erzeugen durch Abgabe von Impulsfolgen, **dadurch gekennzeichnet, dass** die Impulsfolgen auf einer mit Null beginnenden Zeitachse sind
| | |
|---|---|
| Folge 1: | 0 µT für 2-5 Sekunden, |
| Folge 2: | 3-35 µT Grundsignal für 18-22 Sekunden, |
| Folge 3: | 30-120 µT Zusatzsignal für 100-200 Millisekunden, |
| Folge 4: | zwei- bis dreimalige Wiederholung von Folge 2 und 3, |
| Folge 5: | 0 µT für 2-5 Sekunden nach 60-75 Sekunden auf der Zeitachse, |
| Folge 6: | 3-35 µT Grundsignal für 18-22 Sekunden, |
| Folge 7: | 30-120 µT Zusatzsignal für 100-200 Millisekunden, |
| Folge 8: | 3-35 µT Grundsignal für 18-22 Sekunden, |
| Folge 9: | 30-120 µT Zusatzsignal für 100-200 Millisekunden, |
| Folge 10: | 0 µT für 2-5 Sekunden, |
mehrmalige Wiederholung aller Folgen nach 100 bis 130 Sekunden auf der Zeitachse in der angegebenen Reihenfolge,
wobei die Frequenz in den Folgen 2 und 3 im Bereich von 30-35 Hz liegt und die Frequenz in den Folgen 6 bis 9 im Bereich von 8-15 Hz liegt, das Zusatzsignal in allen Folgen wenigstens 20 µT höher ist als das Grundsignal und die Amplituden der Einzelimpulse einer Exponentialfunktion folgen oder bogenförmig an- und absteigenden Hüllkurven mit harmonischem oder anharmonischem Verlauf.

2. Vorrichtung nach Anspruch 1, **dadurch gekennzeichnet, dass** die Felderzeugungseinrichtung eine Spulenmatte ist.

3. Vorrichtung nach Anspruch 2, **dadurch gekennzeichnet, dass** in der Spulenmatte mehrere elektromagnetische Einzelspulen in gleichmäßiger Verteilung über die Gesamtfläche der Matte zur Erzeugung eines flächenhaften Magnetfeldes angeordnet sind.

4. Vorrichtung nach Anspruch 3, **dadurch gekennzeichnet, dass** die Einzelspulen als flächeninhaltsgleiche und/oder -unterschiedliche Leiterschleifen ausgebildet sind.

5. Vorrichtung nach einem der Ansprüche 1 bis 4, **dadurch gekennzeichnet, dass** in den Impulsfolgen das Zusatzsignal in allen Folgen wenigstens 35 µT höher ist als das Grundsignal, insbesondere wenigstens 45 µT höher als das Grundsignal.

6. Vorrichtung nach einem der Ansprüche 1 - 5, **dadurch gekennzeichnet, dass** der Bereich für die Wiederholung aller Folgen auf der Zeitachse 115 bis 125 Sekunden beträgt.

7. Vorrichtung nach einem der Ansprüche 1 bis 4, **dadurch gekennzeichnet, dass** in dem erzeugten pulsierenden elektromagnetischen Feld die Höhe des Grundsignals, die Höhe des Zusatzsignals und die Häufigkeit der Wiederholung der Impulsfolgen in Abhängigkeit von vorhandenen Werten steuerbar sind, ausgewählt unter mikrozirkulatorischen Funktionsmerkmalen, neurovegetativen Merkmalen, immunologischen Merkmalen, Merkmalen der humoralen und zentralnervösen Kreislaufregulation und neurologischen Merkmalen.

## Claims

1. A device for stimulating autoregulative mechanisms of the homeostasis in the organism, comprising a pulse generator, a control device and a field generation means, wherein the pulse generator is configured to generate, together with the control device and the field generation means, a pulsating electromagnetic field by emitting pulse sequences, **characterized in that** the pulse sequences on a time axis starting with zero are:
| | |
|---|---|
| sequence 1: | 0 µT for 2 to 5 seconds, |
| sequence 2: | 3 to 35 µT of basic signal for 18 to 22 seconds, |
| sequence 3: | 30 to 120 µT of additional signal for 100 to 200 milliseconds, |
| sequence 4: | 2- to 3-fold repetition of sequences 2 and 3, |
| sequence 5: | 0 µT for 2 to 5 seconds after 60 to 75 seconds on the time axis, |
| sequence 6: | 3 to 35 µT of basic signal for 18 to 22 seconds, |
| sequence 7: | 30 to 120 µT of additional signal for 100 to 200 milliseconds, |
| sequence 8: | 3 to 35 µT of basic signal for 18 to 22 seconds, |
| sequence 9: | 30 to 120 µT of additional signal for 100 to 200 milliseconds, |
| sequence 10: | 0 µT for 2 to 5 seconds, |
multiple repetition of all sequences after 100 to 130 seconds on the time axis in the order given,
wherein the frequency in sequences 2 and 3 is within the range of 30 to 35 Hz and the frequency in sequences 6 to 9 is within the range of 8 to 15 Hz, the additional signal is at least 20 µT higher than the basic signal in all sequences and the amplitudes of the single pulses follow an exponential function or envelopes rising and falling in an arc-shaped manner with a harmonic or anharmonic progression.

2. The device according to claim 1, **characterized in that** the field generation means is a coil mat.

3. The device according to claim 2, **characterized in that** multiple single electromagnetic coils are arranged in the coil mat, uniformly distributed over the entire area of the mat in order to generate a planar magnetic field.

4. The device according to claim 3, **characterized in that** the single coils are designed as conductor loops having the same and/or differing areas.

5. The device according to any one of claims 1 to 4, **characterized in that** in the pulse sequences the additional signal is at least 35 µT higher than the basic signal in all sequences, in particular at least 45 µT higher than the basic signal.

6. The device according to any one of claims 1 to 5, **characterized in that** the range for the repetition of all sequences is 115 to 125 seconds on the time axis.

7. The device according to any one of claims 1 to 4, **characterized in that** the level of the basic signal, the level of the additional signal and the frequency of repetition of the pulse sequences in the generated pulsating electromagnetic field can be controlled as a function of available values, selected from microcirculatory functional characteristics, neurovegetative characteristics, immunologic characteristics, characteristics of the humoral and central nervous circulatory regulation, and neurological characteristics.

## Revendications

1. Dispositif de stimulation de mécanismes autorégulateurs de l'homéostasie de l'organisme, comprenant un générateur d'impulsions, un appareil de commande et un dispositif générateur de champs, le générateur d'impulsions étant constitué pour produire, conjointement avec l'appareil de commande et le dispositif générateur de champs, un champ électromagnétique pulsé par la délivrance de séquences d'impulsions, **caractérisé en ce que** les séquences d'impulsions, sur un axe de temps commençant par zéro, sont
| | |
|---|---|
| Séquence 1: | 0 µT pendant 2-5 secondes, |
| Séquence 2: | 3-35 µT signal de base pendant 18-22 secondes, |
| Séquence 3: | 30-120 µT signal supplémentaire pendant 100-200 millisecondes, |
| Séquence 4: | deux à trois répétitions des séquences 2 et 3, |
| Séquence 5: | 0 µT pendant 2-5 secondes après 60-75 secondes sur l'axe de temps, |
| Séquence 6: | 3-35 µT signal de base pendant 18-22 secondes, |
| Séquence 7: | 30-120 µT signal supplémentaire pendant 100-200 millisecondes, |
| Séquence 8: | 3-35 µT signal de base pendant 18-22 secondes, |
| Séquence 9: | 30-120 µT signal supplémentaire pendant 100-200 millisecondes, |
| Séquence 10: | 0 µT pendant 2-5 secondes, |
plusieurs répétitions de toutes les séquences après 100 à 130 secondes sur l'axe de temps dans l'ordre de succession indiqué,
la fréquence dans les séquences 2 et 3 se situant dans la plage de 30-35 Hz, et la fréquence dans les séquences 6 à 9 se situant dans la plage de 8-15 Hz, le signal supplémentaire dans toutes les séquences étant supérieur d'au moins 20 µT au signal de base, et les amplitudes des impulsions individuelles suivant une fonction exponentielle ou des enveloppantes montantes et descendantes en forme d'arc avec une allure harmonique ou anharmonique.

2. Dispositif selon la revendication 1, **caractérisé en ce que** le dispositif générateur de champs est un mat de bobines.

3. Dispositif selon la revendication 2, **caractérisé en ce que**, dans le mat de bobines, il est disposé plusieurs bobines individuelles électromagnétiques réparties régulièrement sur la surface totale du mat pour la production d'un champ magnétique plan.

4. Dispositif selon la revendication 3, **caractérisé en ce que** les bobines individuelles sont constituées en tant que boucles conductrices de même aire surfacique et/ou d'aire surfacique différente.

5. Dispositif selon une des revendications 1 à 4, **caractérisé en ce que**, dans les séquences d'impulsions, le signal supplémentaire dans toutes les séquences est supérieur d'au moins 35 µT au signal de base, en particulier est supérieur d'au moins 45 µT au signal de base.

6. Dispositif selon une des revendications 1 à 5, **caractérisé en ce que** la plage pour la répétition de toutes les séquences sur l'axe de temps est de 115 à 125 secondes.

7. Dispositif selon une des revendications 1 à 4, **caractérisé en ce que**, dans le champ électromagnétique pulsé produit, la hauteur du signal de base, la hauteur du signal supplémentaire et la fréquence de répétition des séquences d'impulsions peuvent être commandées en fonction de valeurs existantes sélectionnées parmi des caractéristiques fonctionnelles microcirculatoires, des caractéristiques neurovégétatives, des caractéristiques immunologiques, des caractéristiques de la régulation de circulation humorale et nerveuse centrale, et des caractéristiques neurologiques.
